(19) **Europäisches Patentamt**

**European Patent Office**

**Office européen des brevets**

(11) **EP 0 813 840 B1**

(12) **EUROPÄISCHE PATENTSCHRIFT**

(45) Veröffentlichungstag und Bekanntmachung des Hinweises auf die Patenterteilung:
**15.03.2000 Patentblatt 2000/11**

(51) Int Cl.⁷: **A61B 5/0484**

(21) Anmeldenummer: **97109675.5**

(22) Anmeldetag: **13.06.1997**

(54) **Verfahren und Messanordnung zum Messen von reizevozierten Potentialen des Gehirns**

Method and system for measuring cerebral evoked response potentials

Procédé et système destinés à la mesure de potentiels cérébraux évoqués par stimulation

(84) Benannte Vertragsstaaten:
**DE ES FR GB IT**

(30) Priorität: **17.06.1996 DE 19624133**

(43) Veröffentlichungstag der Anmeldung:
**29.12.1997 Patentblatt 1997/52**

(73) Patentinhaber: **Erich Jaeger GmbH 97204 Höchberg (DE)**

(72) Erfinder: **Dankwart-Eder, Franz, Dipl.-Phys. 97270 Kist (DE)**

(74) Vertreter: **Heusler, Wolfgang, Dipl.-Ing. et al v. Bezold & Sozien Patentanwälte Akademiestrasse 7 80799 München (DE)**

(56) Entgegenhaltungen:
**WO-A-91/19453          WO-A-93/07804
WO-A-96/29928          US-A- 3 513 834**

**Beschreibung**

Die Erfindung betrifft ein Verfahren und eine Meßanordnung zum Messen und/oder Darstellen von reizevozierten Potentialen des Gehirns, insbesondere zum Überwachen der Analgesie-Narkosetiefe, gemäß dem Oberbegriff der unabhängigen Patentansprüche.

Während einer Operation muß mit Sicherheit vermieden werden, daß der Patient aus einer Allgemeinanästhesie erwacht und insbesondere, daß er Operationsschmerz oder andere chirurgische Manipulationen wahrnehmen und postoperativ erinnern kann. Der Schreck eines solchen Erlebnisses kann ein sogenanntes posttraumatisches Streßsyndrom hervorrufen. Es ist daher erwünscht, geeignete Parameter zur Bestimmung der Analgensie-Narkosetiefe zu messen und darzustellen, damit es dem Anästhesisten möglich ist, die Narkose genauer als bisher zu führen und die Belastung des Patienten so gering wie möglich zu halten. Insbesondere müssen ungewollte Wachzustände des Patienten während der Narkose frühzeitig erkennbar sein.

Zur Erfassung von Wachzuständen während der Allgemeinanästhesie sind verschiedene Methoden bekannt. Die wichtigsten sind der sogenannte PRST-Score, der aus Veränderungen von Blutdruck, Herzfrequenz, Schweißsekretion und Tränenfluß errechnet wird, die isolierte Unterarmtechnik, bei der ein Unterarm des Patienten mit einer Blutdruckmanschette oder einer Blutsperre gegen die Betäubung isoliert wird und zur Anzeige von Wachzuständen festgestellt wird, ob der Patient intraoperativ einfachen Kommandos nachkommt, sowie eine EEG-Verarbeitung, bei der zwischen Wach- und Narkosezuständen auftretende Frequenz- und Amplitudenänderungen des Elektroenzephalogramms ausgewertet werden. Der PRST-Score ist aber nicht immer verläßlich, um intraoperative Wachzustände anzuzeigen. Die isolierte Unterarmtechnik kann nur kurzzeitig angewendet werden und ist daher nicht geeignet, motorische Antworten des Patienten während lang dauernder Eingriffe anzuzeigen. Auch das verarbeitete EEG und die daraus abgeleiteten Parameter (Medianfrequenz und spektrale Eckfrequenz) sind nicht optimal zur Anzeige intraoperativer Wachzustände geeignet.

Besser geeignet erscheinen reizevozierte EEG-Signale wie visuell evozierte Potentiale, somatosensorisch evozierte Potentiale und akustisch evozierte Potentiale, die während der Allgemeinanästhesie eine dosisabhängige Unterdrückung erfahren. Besonders ausgeprägt und klar ist dieser dosisabhängige Effekt bei den akustisch evozierten Potentialen mittlerer Latenz. Die akustisch evozierten Potentiale bestehen aus einer Serie positiver und negativer Potentialschwankungen, die an verschiedenen Orten im Verlauf der Hörbahn generiert werden und an der Schädeloberfläche mit Elektroden abgeleitet werden können. Sie reflektieren die Aufnahme, Weiterleitung und Verarbeitung der akustischen Information von der Kochlea durch den Hirnstamm bis zur Hirnrinde. Frühe akustisch evozierte Potentiale werden von Strukturen der peripheren Hörbahn und des Hirnstamms generiert. Sie beweisen die Reiztransduktion und primäre Reiztransmission. Es ist bekannt, daß die frühen akustisch evozierten Potentiale bei der Narkose im Gegensatz zu den dosisabhängig unterdrückten akustisch evozierten Potentialen mittlerer Latenz weitgehend stabil bleiben. und bei der Narkoseüberwachung beide Potentialarten auf einem Bildschirm gemeinsam dargestellt werden können (WO-A-91/ 19453).

Reizevozierte Potentiale sind also von Natur aus gut zur Überwachung der Narkosetiefe und darüber hinaus auch für die Messung sonstiger neurophysiologischer Funktionen geeignet. Aus den Änderungen des zeitlichen Verlaufs der gemessenen Potentiale im Vergleich mit den unverändert bleibenden Potentialen sind Rückschlüsse auf die zu überwachende neurophysiologische Funktion möglich. Es war aber bisher schwierig, diese Eignung in der Praxis zu nutzen. Insbesondere fehlten befriedigende Möglichkeiten zur Messung und Auswertung der auftretenden Potentiale, vor allem wenn die Messung nicht unter Laborbedingungen erfolgt, sondern beispielsweise im Operationssaal. Dort ergeben sich Schwierigkeiten u.a. daraus, daß das Personal nicht mit der Ableitung evozierter Potentiale vertraut ist und daß auch der Anästhesist die gemessenen Kurven nicht ohne weiteres auswerten kann. Die Bedienung der bisher vorgeschlagenen Geräte und die Beurteilung der Ergebnisse ist nur erfahrenen Spezialisten möglich. Ferner müssen die Meßelektroden schnell und einfach anlegbar sein. Andere Probleme ergeben sich durch Störeinstreuung durch Geräte im Operationssaal und durch Einkopplung von Signalen anderer Geräte über die Elektroden. Außerdem können durch Tätigkeiten am Patienten Bewegungsartefakte auftreten.

Besondere Schwierigkeiten ergeben sich bei den für die Messung von Gehirnpotentialen bisher bekannten Verstärkerschaltungen. Sie bestehen üblicherweise aus einem Instrumentenverstärker, der das ankommende Signal (typischerweise 0,5 bis 10 μV) über einen Hochpaß, einen Mittenverstärker, einen Tiefpaß und einen Nachverstärker einem A/D-Wandler zuführt, der mit einer Auswerteeinheit gekoppelt ist. Zwischen dem Patienten und dem die Auswerteeinheit enthaltenden Gerät muß eine Trennstelle z.B. in Form eines Trennverstärkers vom BF- oder CF-Typ vorgesehen sein, um zu verhindern, daß bei externer Spannungsbeaufschlagung unzulässige Ströme in die Meßelektroden fließen können. Diese Trennstelle ist in den bekannten Schaltungen entweder nach der ersten Verstärkerstufe, d.h. nach einer Verstärkung zwischen 10 und weniger als 100 vorgesehen, oder stattdessen nach der vollen Verstärkung von bis zu $10^6$, oder auch erst nach dem A/D-Wandler. Dies hat verschiedene erhebliche Nachteile. Im erstgenannten Fall fließen extrem kleine Signale über Leitungen, die für den treibenden Verstärker eine kapazitive Last darstellen, was Verzerrungen in der Ausgangsstufe und damit eine Verfälschung des Signals zur Folge haben kann. Weiterhin ist aufgrund der kleinen zu übertragenden Spannungen selbst bei niedriger Treiberimpedanz die Störentkopplung begrenzt. Wenn aber die Trennstelle erst nach der vollen Verstärkung vorgesehen ist, ergibt sich zwar eine gute Entkopplung von Einstreuungen, doch läßt die benötigte Anzahl der Bauteile nicht eine gewünschte Miniaturisierung der Schaltung zu. Damit sind die Wege zu den Ableitorten weiter, als für eine optimale Ableitung wünschenswert wäre. Außerdem ist insbesondere bei hohen Verstärkungen (ab etwa 5000) in kompakt aufgebauten Verstärkern die Gefahr der Überkopplung der Ausgangssignale auf den Eingang nicht sicher zu vermeiden. Bei solch hohen Verstärkungen, wie sie bei der Messung von evozierten Potentialen benötigt werden, können Rückkopplungen vom Ausgang des Verstärkers auf seinen Eingang entweder über direkte kapazitive Einkopplung oder über die Versorgungsspannung auftreten. Diese Rückkopplungen wirken sich besonders dann störend aus, wenn die Abschlußimpedanzen (Impedanzen der Elektrodenübergänge) des Eingangsverstärkers hoch oder asymmetrisch sind. Durch eine Rückkopplung ändert sich das Frequenz-Zeit-Verhalten des Verstärkersystems, und es können Instabilitäten entstehen, die bei hoher Bandbreite bis zum Schwingen des Verstärkers führen. Erschwerend kommt hinzu, daß diese Effekte oft das gesamte zu verstärkende Signal nur unmerklich ändern, jedoch gravierende Auswirkungen auf den im Gesamtsignal "versteckten" zu messenden Anteil haben können. So sind z.B. die frühen akustisch evozierten Potentiale (BAEP) mit einer Signalgröße von ca. 1 µVss im spontanen EEG, welches eine Amplitude von 20 µVss bis 50 µVss besitzt, nicht mehr zu erkennen. Nur durch Mittelung kann das BAEP gemessen werden, vorausgesetzt, daß der spontane Anteil des EEG im zeitlichen Mittel nicht mit dem BAEP korreliert und die BAEP-Anteile zeitsynchron nach dem Stimulationssignal auftreten. Diese Voraussetzung ist aber nicht mehr gesichert, wenn sich das Zeitverhalten des Verstärkers ändert oder Nichtlinearitäten mit den unvermeidbaren Intermodulationsverzerrungen auftreten. Die Meßsignale werden verfälscht oder verschwinden durch die Mittelung. Ein weiterer unerwünschter Effekt ist, daß sich durch die Überkopplung auf den Eingang die Gleichtaktunterdrückung verschlechtert. Auch kann bei Gleichtaktstörungen (Netzbrumm, Monitorstörungen, Transienten usw.) das System kurzfristig in einen ungünstigen Arbeitspunkt gefahren werden und dann Instabilitäten zeigen. Diese Instabilitäten sind oft im Einzelsignal kaum zu erkennen, zeigen sich jedoch nach der Mittelung der Signale. Das Signal/Störverhältnis kann sich so verschlechtern, daß die Signale nicht mehr auswertbar sind.

Aufgabe der Erfindung ist, die erwähnten Schwierigkeiten zu vermeiden und ein Verfahren und eine Meßanordnung anzugeben, die eine einfache, zuverlässige und störsichere Messung neurophysiologischer Signale ermöglichen, so daß zuverlässig beurteilbare Messungen durchgeführt werden können, ohne daß zur Bedienung des die Meßanordnung enthaltenden Gerätes und zur Beurteilung der Ergebnisse ein erfahrener Spezialist benötigt wird.

Diese Aufgabe wird durch die in den Patentansprüchen angegebenen Merkmale gelöst.

Gemäß einem ersten Aspekt der Erfindung wird während der Messung und Darstellung der der Funktion entsprechenden Potentiale der zeitliche Verlauf der evozierten Potentiale aus dem Hirnstamm mit einer gegenüber den Informationsignalen vergrößerten Zeitauflösung dargestellt.

Wesentlich ist, daß die Darstellung der Vergleichskurve bzw. sonstigen Kurven (d.h. Bereitstellung der Vergleichs- bzw. sonstigen Kurvenwerte) gleichzeitig während der Messung und ggf. Darstellung der Informationssignale erfolgt, und zwar ebenso wie die Darstellung der Informationskurve selbsttätig beispielsweise auf einem Monitor unter Steuerung des in der Auswerteeinrichtung für die gemessenen Potentiale enthaltenen Rechners.

Durch die Erfindung wird erreicht, daß auch unter ungünstigen Bedingungen z.B. bei Operationen stets eine einfache und zuverlässige Auswertung der gemessenen Signale möglich ist. Dies ist besonders vorteilhaft zur Überwachung der Narkosetiefe. Da sich die Kurve der evozierten Potentiale aus dem Hirnstamm durch die Narkose nicht verändert und durch den vergrößerten Zeitmaßstab gut auswertbar ist, kann sie als Maß für die Güte der Ableitung der gemessenen Informationssignale dienen. Verschlechtert sich die Vergleichskurve, so kann daraus auf verschlechterte Ableitbedingungen geschlossen werden.

Eine besonders vorteilhafte und informative Auswertung der gemessenen Signale wird dadurch ermöglicht, daß die evozierenden Reize periodisch oder stochastisch verteilt wiederholt werden und jeweils nach einer vorbestimmten Zeit oder Anzahl von Meßperioden die aktuelle Informationskurve dargestellt wird, welche über einen wesentlich größeren Zeitraum oder eine wesentlich größere Anzahl von vorangegangenen Meßperioden gemittelt wurde. Hierbei ist es zweckmäßig, daß bei der Mittelung über die vorbestimmte Anzahl von Meßperioden jeweils die Mittelwerte der während einer wesentlich geringeren Zahl von Meßperioden gemessenen Signale gebildet und selbsttätig miteinander verglichen werden, und daß diese Mittelwerte ihrerseits gemittelt werden, wobei von der Mehrzahl der übrigen Mittelwerte wesentlich abweichende Mittelwerte vernachlässigt werden. Die Möglichkeit, Untergruppen der Mitteilung miteinander zu korrelieren, verbessert die Möglichkeit, Artefakte zu erkennen und zu eliminieren.

Besonders zweckmäßig kann es auch sein, wenn der zeitliche Verlauf von Änderungen eines charakteristischen Peaks der Potentiale mittlerer Latenz (MLAEP) und/oder der Latenz dieses Peaks und/oder des selbsttätig errechneten Quotienten aus diesen beiden Faktoren dargestellt wird.

Ferner ist es zweckmäßig, daß gemeinsam mit dem zeitlichen Verlauf der Meßwerte der zeitliche Verlauf eines von der Auswerteeinrichtung errechneten veränderlichen Meßgerätefaktors dargestellt wird, wodurch zusätzliche Sicherheit bezüglich der Qualität der Messung erreicht wird.

Für die Auswertung der evorzierten Potentiale müssen diese typisch um Faktoren zwischen $10^5$ und $10^6$ verstärkt werden. Gemäß einer Weiterbildung der Erfindung ist die Gesamtverstärkung durch den zwischen die Elektrodenanordnung der Meßanordnung und die oben erwähnte Trennstufe geschalteten Verstärkerteils wesentlich größer als 100, jedoch geringer als $10^4$, während für die restliche Verstärkung der Potentiale der zwischen die Trennstufe und die Auswerteeinheit geschaltete Nachverstärker sorgt. Wenn z.B. die Verstärkung vor der Trennstelle zwischen 1000 und 4000 beträgt, ist also eine Nachverstärkung der evozierten Potentiale um mehr als 25 und typisch zwischen 100 und 1000 erforderlich. Durch diese Maßnahme ergibt sich ein optimaler Kompromiß zwischen der theoretisch erreichbaren höchsten Gleichtaktunterdrückung (möglichst hohe Verstärkung vor der Potentialtrennung) und einer minimalen Überkopplung auf den Eingang (möglichst niedrige Verstärkung vor der Potentialtrennung). Die Gleichtaktunterdrückung kann bei Verstärkungen von etwa 1000 bereits 150 dB betragen. Gleichzeitig wird durch die Potentialtrennung nach etwa 1000-facher Verstärkung der Eingangskreis vom Ausgangskreis soweit entkoppelt, daß eine Instabilität des Verstärkers nicht mehr zu befürchten ist. Das Ausgangssignal mit der größten Amplitude ist dann von der Versorgungsspannung des Eingangskreises entkoppelt. Gleichzeitig wirkt das Ausgangssignal auf den Eingangskreis nur noch wie ein Gleichtaktsignal. (Eine weitere Verbesserung ist zu erzielen, wenn die Masse des Ausgangskreises auf Erdpotential liegt.) Hierdurch wird insbesondere auch die Bedienung der Meßanordnung vereinfacht und erleichtert.

Die beschriebene Meßanordnung hat darüberhinaus den Vorteil, daß die benötigte Verstärkerschaltung optimal miniaturisiert werden kann, ohne daß dadurch Nachteile wie lange Leitungen zu den Ableitorten, Signalverfälschungen, ungenügende Störentkopplung, Einstreuungen von Störsignalen usw. in Kauf genommen werden müssen.

Eine weitere Verbesserung der Zuverlässigkeit ergibt sich, wenn in Weiterbildung der Erfindung an die Eingänge der Verstärkerschaltung eine Impedanzmeßschaltung zum Messen der Elektrodenimpedanzen angeschlossen wird, die bei Überschreiten eines maximalen Wertes und/oder einer bestimmten Differenz zwischen z.B. der aktiven Elektrode und der Referenzelektrode ein Alarmsignal erzeugen kann. Falsche Elektrodenimpedanzen, die im wesentlichen von dem Übergang zum Kopf des Patienten abhängen, können bei dem erfindungsgemäß ausgeführten Verstärker zu Meßfehlern führen.

Wie schon erwähnt wurde, eignet sich die Erfindung nicht nur für die Bestimmung der Narkosetiefe, sondern kann zur Messung beliebiger, z.B. auch visuell evozierter Potentiale angewendet werden. Im folgenden wird die Erfindung an dem bevorzugten Beispiel der Messung akustisch evozierter Potentiale zur Überwachung der Narkosetiefe erläutert. In der Zeichnung zeigen:

Fig. 1 den zeitlichen Verlauf der bei einem wachen Patienten gemessenen akustisch evozierten Potentialen;

Fig. 2 erfindungsgemäß dargestellte Kurven der gemessenen Potentiale gemäß Fig. 1;

Fig. 3 die dosisabhängige Änderung von MLAEP-Kurven;

Fig. 4, 5 und 6 verschiedene Kurven zur Erläuterung der Erfindung;

Fig. 7 eine Prinzipdarstellung der Verstärkerschaltung einer erfindungsgemäßen Meßanordnung;

Fig. 8 eine Schaltungsanordnung zur Messung der Elektrodenimpedanzen der Meßanordnung;

Fig. 9 eine zweckmäßige Ausgestaltung der Eingangsstufe der Verstärkerschaltung nach Fig. 2.

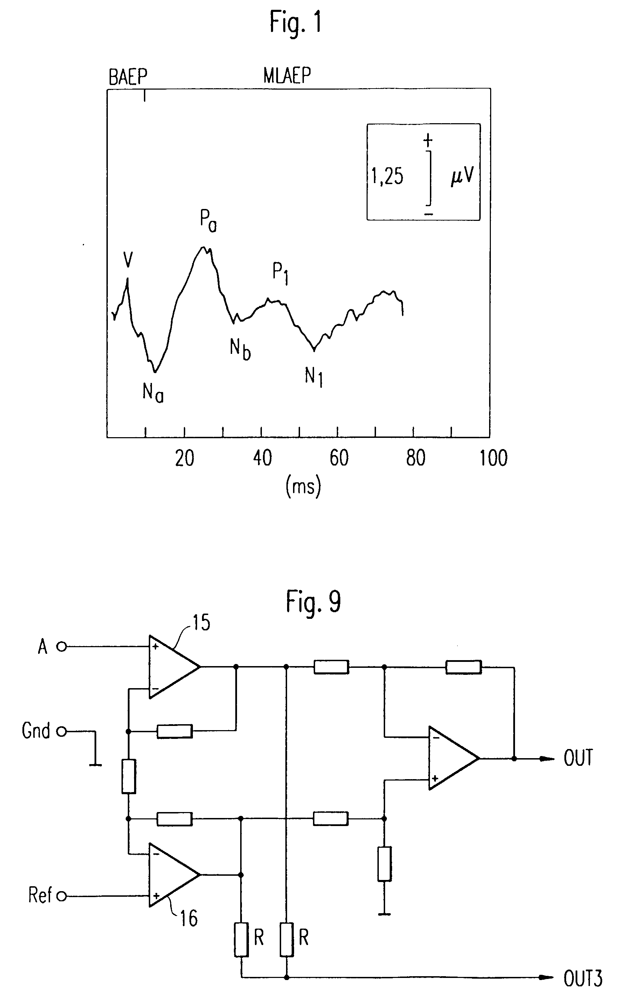

Aus dem zeitlichen Verlauf vor und während einer Operation gemessener akustisch evozierter Potentiale lassen sich bekanntlich wichtige Informationen über den Narkosezustand eines Patienten ableiten. Der akustische Reiz kann im einfachsten Fall ein Klick sein und wird in der Praxis dem Patienten über Kopfhörer mit einer geeigneten Wiederholfrequenz z.B. in der Größenordnung von 9 oder 10 Hz appliziert. Gemessen werden insbesondere die evozierten Potentiale bis etwa 100 ms nach Reizbeginn, wobei zwischen den frühen akustisch evozierten Potentialen aus dem Hirnstamm (Brainstem Acoustic Evoked Potentials oder BAEP) und den akustisch evozierten Potentialen mittlerer Latenz (Midlatency Acoustic Evoked Potentials oder MLAEP) zu unterscheiden ist. Fig. 1 zeigt den Verlauf der bei einem wachen Patienten gemessenen Potentiale. Bei einem um 60 dB überschwelligen Reiz liegen die ersten fünf Peaks der BAEP bei Gesunden zwischen etwa 1,5 ms und 6 ms und die Peaks $N_a$ bis $N_1$ (N und P bedeuten negativ bzw. positiv) der MLAEP zwischen etwa 10 ms und 100 ms.

Die MLAEP werden, wie aus der Literatur bekannt ist, bei der Narkose dosisabhängig unterdrückt. Die Amplitude der MLAEP-spezifischen Peaks sinkt mit steigender Narkosetiefe, während die Latenzen verlängert sind. Im Gegensatz zum Roh-EEG ist es damit möglich, bereits mehrere Minuten vor einer Aufwachreaktion diese Änderung der Narkosetiefe zu erkennen. Damit lassen sich rechtzeitig Maßnahmen zur Vermeidung von ungewollten Wachzuständen während der Narkose ergreifen. Für eine richtige und zuverlässige Ableitung und Auswertung der gemessenen MLAEP-Signale bedarf es aber einer Vergleichsmöglichkeit, die bei dem hier beschriebenen Verfahren durch die unabhängig von der Narkosetiefe weitgehend gleichbleibenden frühen BAEP-Signale realisiert wird.

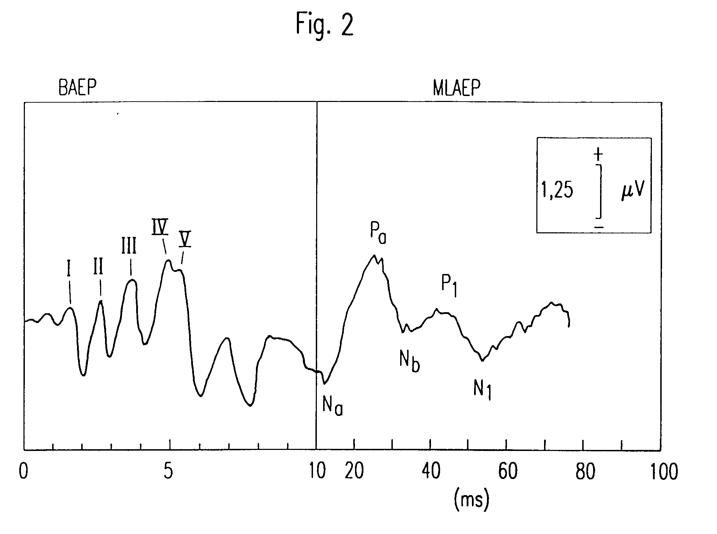

Da das Ergebnis der Messung der nur kurzzeitig nach Reizbeginn auftretenden BAEP-Signale schwierig auswertbar ist, wie aus Fig. 1 ersichtlich ist, wird die BAEP-Kurve erfindungsgemäß mit vergrößerter Zeitauflösung und vergrößerter Amplitudenauflösung dargestellt. Ein Beispiel hierfür zeigt Fig. 2, wonach die BAEP-Kurve so "gedehnt" wird, daß sie zwischen 0 und 10 ms beispielsweise eine ähnliche Breite hat wie die MLAEP-Kurve zwischen 20 und 100 ms, also wesentlich breiter ist als bei der Aufzeichnung mit derselben Zeitauflösung wie die MLAEP-Kurve (Fig. 1).

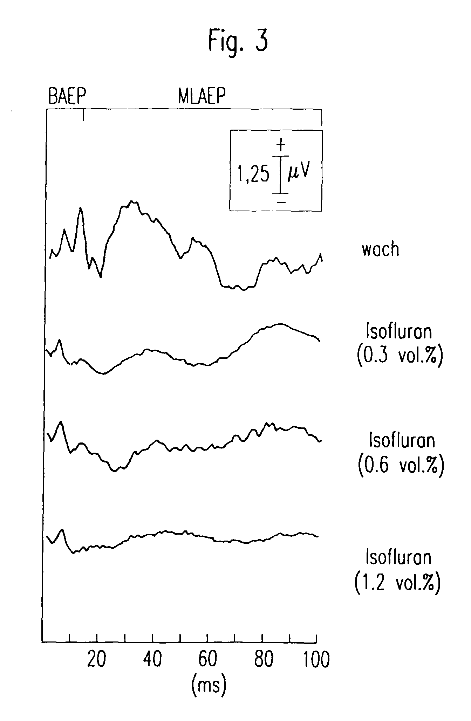

Fig. 3 zeigt am Beispiel zunehmender Konzentrationen von Isofluran die dosisabhängige Zunahme der Latenzen und Abnahme der Amplituden der MLAEP.

Die Kurven werden in an sich üblicher Weise im Rechner der Auswerteeinheit gebildet und beispielsweise auf einem Monitor dargestellt. Vorzugsweise werden in der Praxis nicht nur die während der Operation am narkotisierten Patienten gemessenen aktuellen BAEP- und MLAEP-Kurven dargestellt, sondern sowohl die im Wachzustand vor der Operation aufgenommenen Kurven (Fig.2) als auch weitere Kurven wie die sich in regelmässigen Zeitabständen nacheinander, beispielsweise in Abständen von jeweils einer Minute ergebenden letzten fünf BAEP- und MLAEP-Kurven, so daß ein mittelfristiger Entwicklungstrend beispielsweise der jeweils letzten fünf Minuten vor den aktuellen Kurven erkennbar ist. Alle genannten Kurven können gleichzeitig dargestellt werden und z.B. etwa der Darstellung der Fig. 3 entsprechen, wobei unter der als Referenzkurve dienenden Wachzustandskurve als unterste Kurve die jeweils aktuelle Meßkurve und dazwischen die während einer vorhergehenden Zeitspanne, z.B. jeweils während der letzten 10 Minuten gemessenen Kurven dargestellt werden. Während die unterste Kurve den nachfolgend beschriebenen gleitenden Mittelwert darstellt, lassen die mittleren Kurven einen mittelfristigen Trend von Änderungen der Meßsignale erkennen.

Wegen der geringen Größe der Meßsignale und der überlagerten spontanen EEG-Potentiale müssen sie vom Rechner der Auswerteeinheit gemittelt werden. Für die BAEP-Kurve sind mindestens 1000 Mittelungen notwendig, bevor sich eine gut auswertbare Information ergibt, noch besser sind beispielsweise 2000 oder 4000 Mittelungen, während für die MLAEP-Kurve 500 oder besser 1000 genügen. Bei einer Stimulationsfrequenz von etwa 10 Hz und 1000 Mittelungen steht damit ungefähr alle zwei Minuten jeweils ein Meßsignal zur Verfügung. Da aber bei solchen relativ großen Zeitabständen jeweilige Änderungen der Meßsignale nur in entsprechend großen Rastern aufgezeichnet und ausgewertet werden können, wird der Meßabstand dadurch verringert, daß die aktuellen Meßkurven, insbesondere die aktuelle Informationskurve im "gleitenden zeitlichen Mittel" ermittelt und dargestellt werden. Der gleitende Mittelwert wird dadurch gebildet, daß nach jeweils z.B. 100 Mittelungen das Mittel der letzten z.B. 1000 Werte gebildet und dargestellt wird, und zwar vorzugsweise der letzten 1000 artefaktfreien Werte (Artefakte sind vom Patienten oder durch Störungen außerhalb des Gerätes hervorgerufene Fehler).

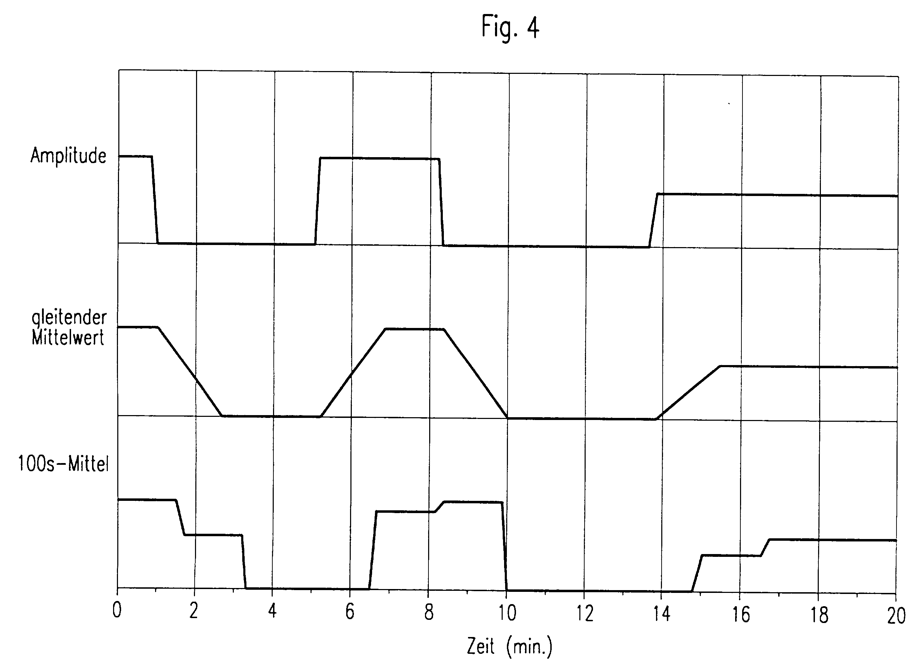

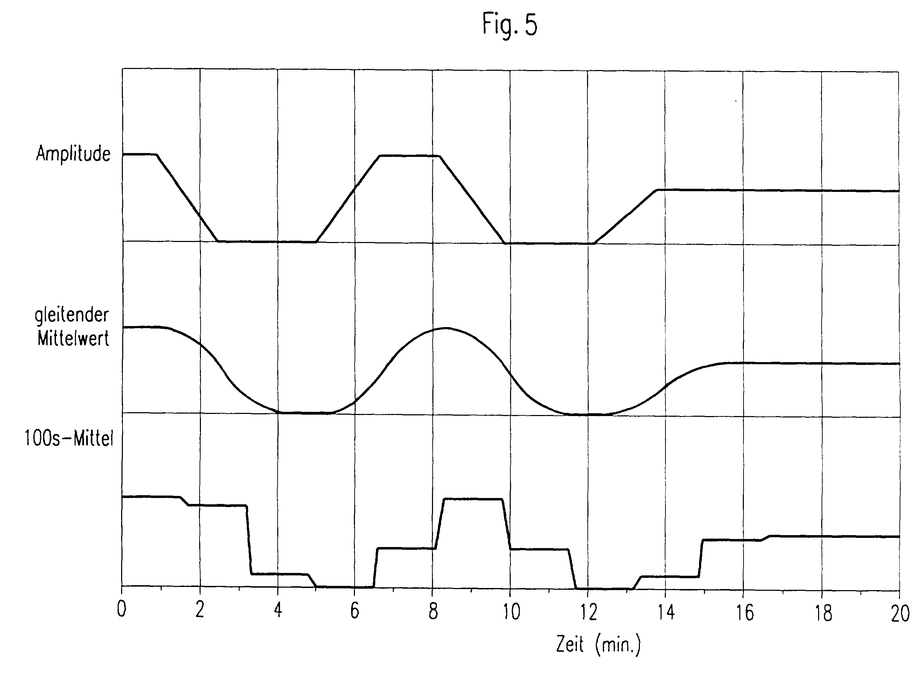

Der Effekt dieser Maßnahme auf die Trenddarstellung der Amplituden der Meßsignale ist schematisch in Fig. 4 bei sprunghafter Amplitudenänderung und in Fig. 5 bei einer zeitlich linearen Änderung der Amplitude dargestellt. Die jeweils obere Kurve stellt den zeitlichen Verlauf der tatsächlichen Amplitude dar. Darunter ist der bei einer gleitenden Mittelwertbildung erkannte Trend dargestellt, während die untere Kurve den Trend bei einer einfachen Mittelung über jeweils 100 Sekunden zeigt. Hieraus ist ersichtlich, daß der gleitende Mittelwert besser den wahren Trend des Meßsignals wiedergibt und früher auf Änderungen des Meßsignals reagiert.

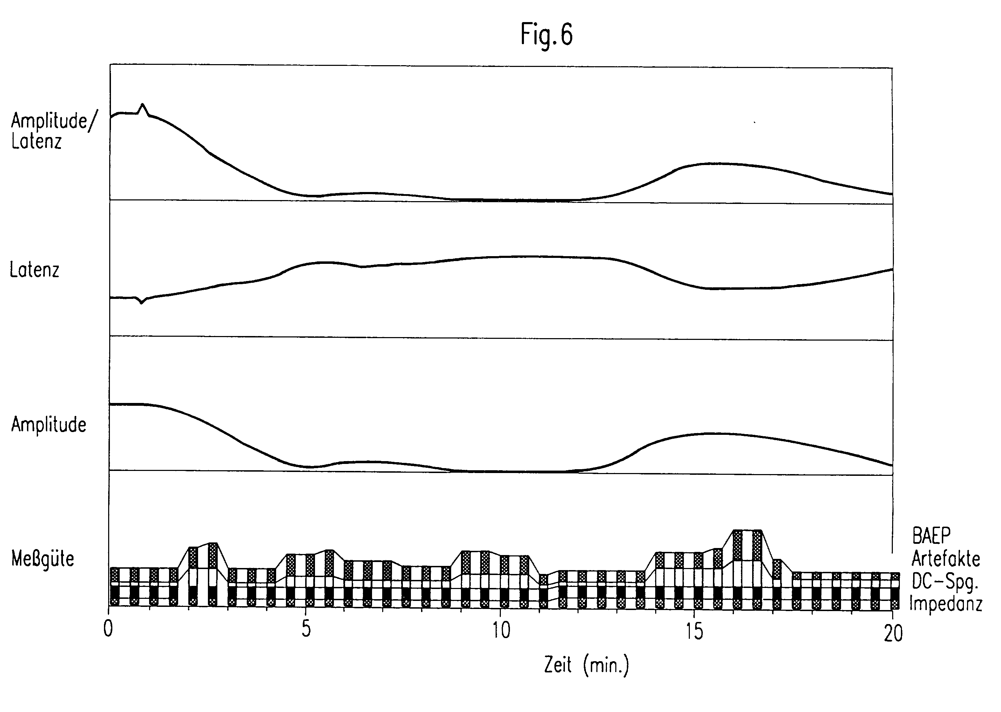

Ein Entwicklungstrend kann auch selbsttätig als gesonderte Graphik dargestellt werden, wie beispielsweise in Fig. 6 gezeigt ist. In diesem Fall ist es möglich, die automatisch ausgewerteten Amplituden und Latenzen und/oder sonstige Informationen als Trend darzustellen. Als Beispiel hierfür zeigt die obere Kurve der Fig. 6 die zeitliche Änderung des Quotienten aus einer bestimmten Amplitude, hier des Peaks $N_b$ (vgl. Fig. 1) und der zugehörigen Latenz, während darunter die Kurven der Latenz bzw. Amplitude selbst dargestellt sind. Unter den relevanten Meßparametern sind die Amplitude und Latenz des $N_b$-Peaks besonders aussagekräftig. Andere charakteristische Informationen liefert beispielsweise auch das Produkt aus der Latenz und der Amplitudendifferenz bestimmter Peaks.

Wie die unterste Graphik in Fig. 6 zeigt, besteht ferner die Möglichkeit, vorzugsweise in bildlicher Relation zu den dargestellten Meß- und/oder Trendkurven auch den zeitlichen Verlauf einer Information über die Güte der Ableitung der Meßsignale darzustellen. Die Güte der Ableitung wird bei dem dargestellten Beispiel aus der Größe der Elektrodenimpedanz, der Größe der Elektroden-Gleichspannung, der Artefakthäufigkeit und der Güte der BAEP-Messung errechnet. Jede dieser Komponenten wird gemessen, und je nach dem Meßergebnis wird ihnen ein bestimmter Bewertungsfaktor zugeordnet, der bei der Berechnung der Meßgüte berücksichtigt wird. Beispielsweise erhalten die Elektrodenimpedanz für gemessene Werte zwischen 0 und mehr als 30 k , die Elektroden-Gleichspannung für gemessene Werte zwischen 0 und mehr als 100 mV, die Artefakthäufigkeit für den Bereich zwischen 0 und mehr als 9% sowie die BAEP-Qualität jeweils einen Bewertungsfaktor zwischen 1 und 10. Die BAEP-Qualität kann aus verschiedenen Faktoren errechnet werden, insbesondere aus Änderungen in Korrelation zur Wachzustandskurve, der Sicherheit der Erkennung des Peaks V (vgl. Fig. 1) und Abweichungen zwischen geglätteten und ungeglätteten Kurven. Die Bewertungsfaktoren können beispielsweise in der Trenddarstellung entweder einzeln oder als ggf. gewertetes Produkt erscheinen.

Ferner kann es zweckmäßig sein, daß in der Verbindung mit den dargestellten Kurven selbsttätig errechnete Zeit- und/oder Amplitudenmarkierungen und/oder sonstige Zahlenwerte angezeigt oder dargestellt werden.

Der Erfolg der beschriebenen Meßmethode ist in erheblichem Maße von der Meßsicherheit abhängig. Insbesondere bei dem betrachteten Fall der Narkoseüberwachung ist der Anästhesist meist nicht in der Lage, die Meßkurven selbst zu beurteilen. Wenn die Meßsignale verfälscht werden, beispielsweise durch Artefakte, könnte er falsche Rückschlüsse ziehen. Zur selbsttätigen Überwachung der Meßsicherheit erfolgt daher bei dem hier beschriebenen System ein Überprüfen der Elektrodenimpedanzen in regelmässigen Abständen, Überprüfen der Elektroden-Gleichspannung, Artefakterkennung und Unterdrückung, sowie ein Referenzvergleich.

Das Überprüfen der Elektrodenimpedanzen ist bei der Langzeit-Ableitung biologischer Signale an sich üblich. Die Messung erfolgt bei einer festen Frequenz von beispielsweise 100 Hz oder mit Gleichstrom, und beim Überschreiten eines Grenzwertes wird ein Alarmsignal erzeugt.

Auch die regelmässige Messung der Elektroden-Gleichspannung ist an sich bekannt. Sie ist bei dem hier beschriebenen System notwendig, weil sich bei schlechten Elektroden oder unterschiedlichen Elektrodenmaterialien für die aktive Elektrode und die Referenzelektrode relativ hohe Gleichspannungen (bis zu 500 mV) an den Elektroden bilden können. Dies hat zur Folge, daß bei Bewegungen der Elektrodenkabel und/oder der Elektroden und bei den damit verbundenen Kapazitätsänderungen zwischen den Kabeln sowie Veränderungen des Elektrode/Haut-Kontakts Spannungen erzeugt werden, die dem zu messenden Signal überlagert werden. Die hierdurch entstehenden Bewegungsartefakte können die Ableitung der Signale erheblich stören. Werden diese Gleichspannungen zu groß, so können sie zudem bei gleichspannungsgekoppelten Verstärkern diese übersteuern, so daß das Signal verformt oder verkleinert wird oder ganz verschwindet.

Wie oben schon erwähnt wurde, sollen bei der Mittelwertbildung nur artefaktfreie Werte berücksichtigt werden. Oft werden nämlich durch Signale, die im Übertragungsbereich des Meßsignals liegen, aber nicht seiner Signalform entsprechen, die Messungen so stark verfälscht, daß die Auswertung fehlerhaft wird. Beispielsweise kann der tatsächliche Verlauf der gemessenen Potentiale durch ein überlagertes 50 Hz-Netzstörsignal verfälscht werden.

Hierdurch verursachte Meßfehler können durch eine Frequenzanalyse vermieden werden, die besonders effektiv bei Störungen konstanter Frequenz ist, und/oder durch eine Kreuzkorrelation zu einer bekannt guten Kurve, wofür sich bei dem hier beschriebenen System das dosisunabhängige konstante BAEP-Signal besonders gut eignet. Im Falle der dosisabhängigen Signale wie z.B. der MLAEP ist eine Frequenzanalyse sowie eine Kreuzkorrelation zwischen Teilsummensignalen vorzuziehen. Der selbsttätige Vergleich der Frequenzinhalte und/oder die Kreuzkorrelation zu Referenz- und/oder Teilsummensignalen ist also ein weiteres wichtiges Beurteilungskriterium für die Meßgüte.

Darüberhinaus kann die BAEP-Kurve als Referenz zur Beurteilung der Ableitebedingungen verwendet werden. Die Einkopplung von Störsignalen sind in der BAEP-Kurve sofort zu erkennen. Der Vergleich der aktuell gemessenen BAEP-Kurve mit einer bekannt guten Kurve wie beispielsweise der vor der Operation gemessenen Kurve läßt eine Beurteilung der Ableitebedingungen und damit der Meßgüte zu. Dieser Vergleich kann entweder visuell oder selbsttätig durch das Verarbeitungssystem erfolgen.

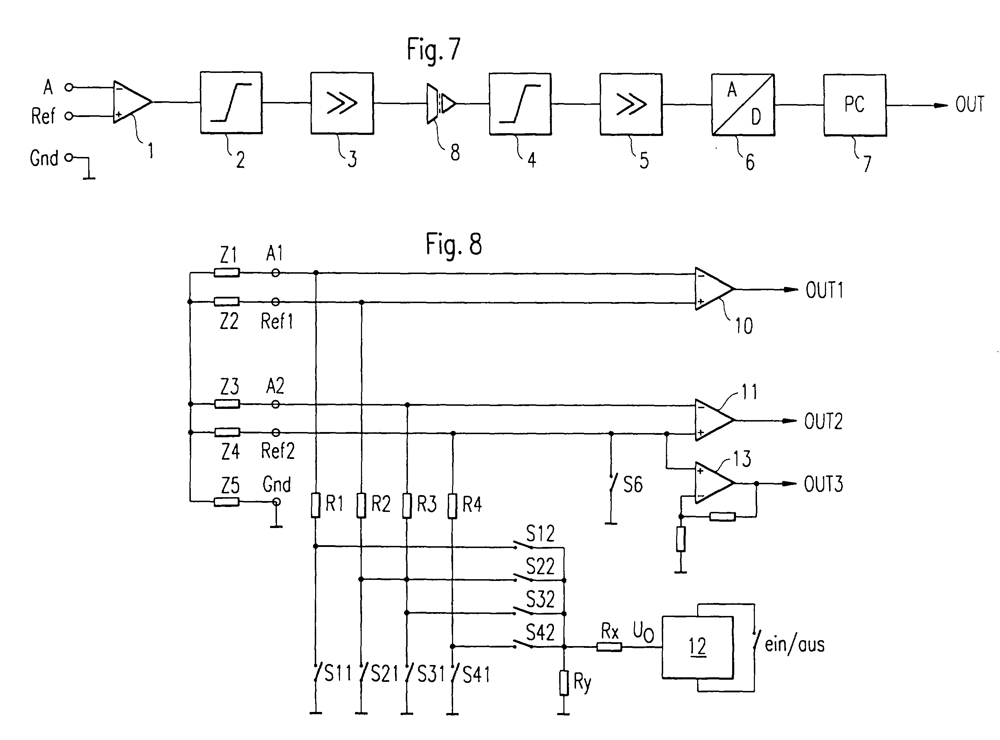

Die gute und zuverlässige Ableitung der darzustellenden Signale hängt im übrigen wesentlich von der eingesetzten Verstärkertechnik ab. Die Verstärker müssen bei der Ableitung der evozierten Potentiale, also von Spannungen in der Größenordnung von 0,5 bis 10 $\mu V$, die zudem durch die spontanen EEG-Potentiale überlagert sind, möglichst linear, intermodulationsfrei und rauscharm sein und eine hohe Gleichtaktunterdrückung aufweisen. Die Zuleitungen vom Ableiteort zum Verstärker müssen so kurz wie möglich sein und durch geeignete Maßnahmen unempfindlich gegen einstreuende Störpotentiale sein. Eine Verstärkerschaltung, die diesen Anforderungen entspricht, ist in Fig. 7 dargestellt. Sie enthält eine als Eingangsstufe dienende Instrumentenverstärkerstufe 1, die das ankommende Signal verstärkt und es über einen Hochpaß 2, einen Mittenverstärker 3, einen Tiefpaß 4 und einen Nachverstärker 5 über einen A/D-Wandler 6 einer üblicherweise durch einen PC oder sonstigen Rechner gebildeten Auswerteeinheit 7 zuführt. Insoweit entspricht der Verstärker auf dem vorliegenden Fachgebiet bekannten Schaltungen. Erfindungsgemäß ist aber die zur galvanischen und körperlichen Trennung zwischen dem patientennahen Verstärkerteil und dem gerätenahen Verstärkerteil zum Schutz des Patienten notwendige Trennstufe 8 an den Ausgang der dem Hochpaß 2 nachgeschalteten Verstärkerstufe 3 angeschlossen, wodurch sich die weiter oben schon erläuterten Vorteile ergeben. Die Verstärkung der Vorverstärkereinheit mit der Stufe 3 wird so eingestellt, daß bei einem Full-Scale-Eingangsbereich von z.B. 10 µV das Full-Scale-Ausgangssignal immer mindestens einige Millivolt beträgt. Diese Größe ist so gewählt, daß einerseits durch Übernahmeverzerrungen der Verstärkerstufe 3 bedingte Signalverzerrungen unterhalb der für die jeweiligen Signale relevanten Grenzen bleiben, während andererseits ein ausreichend hohes Signal/Störverhältnis gewährleistet ist und das Ausgangssignal nicht wesentlich auf die eingangsseitigen Signalleitungen einkoppelt. Anders gesagt soll die Gesamtverstärkung des zwischen die Elektrodenanordnung und die Trennstufe 8 geschalteten Verstärkerteils wesentlich größer als 100, jedoch geringer als $10^4$ sein und vorzugsweise zwischen ungefähr 1000 und 2000 bis höchstens 4000 betragen.

Die Trennstufe 8 ist eine aktive Schaltung und kann z.B. ein mit kapazitiver Kopplung und Signalmodulation arbeitender Isolationsverstärker sein, der als integrierte Schaltung im Handel erhältlich ist (Typ ISO der Firma Burr Brown). Stattdessen kann auch ein optoelektronischer Entkopplungsverstärker oder eine mit Modulation und Demodulation des Signals vor bzw. nach einer magnetischen Entkopplung arbeitende Schaltung verwendet werden (z.B. Isolationsverstärker AD 215 der Fa. Analog Devices).

Die Elektrodenanordnung besteht in dem dargestellten einfachen Fall aus den beiden Meßelektroden A und Ref, die an die beiden Eingänge der Eingangsstufe 1 geschaltet sind, und einer an Masse liegenden Elektrode Gnd. Die Meßelektrode A wird als aktive Elektrode bezeichnet, die dem Patienten an einer Stelle angelegt wird, wo die im Gehirn entstehenden interessierenden Potentiale am besten abgeleitet werden können. Die Elektrode Ref wird als Referenzelektrode an einer Stelle angelegt, wo die Potentiale keine Auswirkungen haben. Die Masseelektrode Gnd wird an einer beliebigen weiteren Stelle angebracht.

Der Hochpaß 2 und der Tiefpaß 4 werden so eingestellt, daß man aus dem Frequenzspektrum der BAEP- und MLAEP-Kurven alle gewünschten Informationen erhält. Hierbei besteht die Möglichkeit, beide Signale im gleichen Frequenzband, z.B. zwischen 10 Hz und 3 kHz aufzunehmen und zu übertragen, d.h. beide Meßsignale werden optimal übertragen. In diesem Fall wird also der Hochpaß auf 10 Hz und der Tiefpaß auf 3 kHz eingestellt. Wenn beide Signale im selben Meßkanal übertragen werden, ergibt sich geringer Verstärkeraufwand. In der Auswerteeinheit 7 werden die Meßsignale dann so gefiltert, daß die Wiedergabe der einzelnen Komponenten, also der BAEP- bzw. MLAEP-Signale optimal ist. Dies ist für die BAEP z.B. der Frequenzbereich von 100-3000 Hz und für die MLAEP der Bereich von 10-200 Hz. Durch digitales Filtern und sonstige digitale Nachbearbeitung ergeben sich gute Meßergebnisse.

Eine andere Möglichkeit, die eine optimale Vorverarbeitung der Meßsignale ermöglicht, besteht darin, die BAEP und MLAEP über zwei getrennte Meßkanäle aufzunehmen und zu verarbeiten.

Die einwandfreie Ableitung, Verstärkung und Darstellung der gemessenen Potentiale ist nur dann möglich, wenn die dem Patienten angelegten Elektroden die richtigen Impedanzwerte haben. Durch die bei der hier beschriebenen Meßanordnung vorgesehene Impedanzmeßschaltung wird daher eine weitere Verbesserung gegenüber üblichen Schaltungen erreicht.

Zur Impedanzmessung werden die Eingangsabschlußwiderstände des Verstärkers zur Einspeisung eines Meßstromes genutzt. In Fig. 8 ist ein bevorzugtes Ausführungsbeispiel in Form einer 2-Kanal-Anordnung dargestellt, in der die Impedanzen aller Elektroden von zwei voneinander getrennten Verstärkerschaltungen 10 bzw. 11 gemessen werden können. Mit Z1 und Z2 sind die Impedanzen der Meßelektroden A1 und Ref1 des Verstärkers 10 bezeichnet, während Z3 und Z4 die Elektrodenimpedanzen der Elektroden A2 und Ref2 des Verstärkers 11 sind. Z5 ist die Elektrodenimpedanz der gemeinsamen an Masse geschalteten Elektrode Gnd. Die beiden Eingänge jedes der beiden Verstärker sind über je einen Eingangsabschlußwiderstand R1, R2, R3 bzw. R4 an Masse schaltbar. Zwischen Masse und jedem Eingangsabschlußwiderstand ist aber ein erster Schalter S11, S21, S31 bzw. S41 angeordnet, und je ein zweiter Schalter S12, S22, S32 bzw. S42 befindet sich zwischen der Verbindung der Eingangsabschlußwiderstände mit den ersten Schaltern und einem Meßstromgenerator 12, wie aus der Zeichnung ersichtlich ist. Die Spannung des Generators 12 kann entweder direkt oder darstellungsgemäß über einen durch die Widerstände $R_x$ und $R_y$ gebildeten Spannungsteiler abgegriffen werden. Der Spannungsteiler $R_x/R_y$ ist vorzuziehen, da eventuell vorhandene Störungen aus dem abgeschalteten Generator im Meßbetrieb besser von den Signaleingängen der Verstärker ferngehalten werden.

Die Elektrodenimpedanzen liegen bei einer Meßfrequenz (des gemessenen Signals) von z.B. 100 Hz in der Regel unter 100 k (höhere Impedanzen sind für Ableitungen von evozierten Potentialen unzweckmäßig), während die Größe der Eingangsabschlußwiderstände üblicherweise zwischen 10 und 100 M beträgt.

Zur Messung beispielsweise der Impedanz der aktiven Elektrode A1 des ersten Kanals wird der Schalter S11 geöffnet, der Schalter S12 geschlossen und der Meßstromgenerator 12 eingeschaltet.

Über den Widerstand R1 fließt nun in den Eingang an der aktiven Elektrode A1 der Meßstrom

$$I_1 = U_0/(R1 + Z1) \approx U_0/R1.$$

Der Meßstrom erzeugt über Z1 einen Spannungsabfall von $I_1 . Z_1$, der durch den Verstärker 10 verstärkt wird. Damit kann über die nachgeschaltete Elektronikanordnung die Elektrodenimpedanz bestimmt werden. In ähnlicher Weise wird die Messung der Elektrodenimpedanzen Z2, Z3 und Z4 durchgeführt.

Zur Messung der Impedanz Z5 der an Masse liegenden Elektrode Gnd sind drei verschiedene Möglichkeiten zweckmäßig, nämlich Kurzschließen eines Eingangs nach Masse, Abgreifen der Spannung an Z5 über einen getrennten Verstärker oder Abgreifen der Spannung durch einen Teil eines der Verstärker 10 oder 11.

Gemäß der erstgenannten Möglichkeit wird einer der Eingänge A1, Ref1, A2 oder Ref2 über einen weiteren Schalter S6 nach Masse gezogen. Bei dem in Fig. 8 dargestellten Beispiel ist der Schalter S6 an den mit der Referenzelektrode Ref2 verbundenen Eingang des Verstärkers 11 angeschlossen. In diesem Fall kann der Meßstrom in die Elektrode A1 oder Ref1 oder in beide Elektroden gleichzeitig eingespeist werden. Der Verstärker 11 kann nun den Spannungsabfall über der Elektrodenimpedanz Z5 messen. Denkbar ist auch die Einspeisung in die aktive Elektrode A2, wobei der Spannungsabfall an dem an dieser Elektrode angeschlossenen Eingang des Verstärkers 11 nun proportional zur Summe der Impedanzen Z3 und Z5 ist.

Gemäß der zweitgenannten Möglichkeit kann die Spannung über Z5 durch einen in der dargestellten Weise parallel zu einem der Verstärker 10 oder 11 geschalteten zusätzlichen Operationsverstärker 13 abgegriffen und verstärkt werden. Bei dem in Fig. 8 dargestellten Beispiel ist dieser Operationsverstärker 13 mit seinem einen Eingang an den mit der Referenzelektrode Ref2 verbundenen Eingang des Verstärkers 11 angeschlossen. Die Einspeisung kann in einen oder mehrere der Eingänge an den Elektroden A1, Ref1 oder A2 erfolgen. Für den Abgriff der Spannungen an einem der anderen Eingänge wird entsprechend dem dargestellten Beispiel an einem oder mehreren der verbleibenden Eingänge der Meßstrom eingespeist.

Gemäß der dritten Möglichkeit kann der Abgriff der Spannung über Z5 im Verstärker 10 oder 11 selbst erfolgen, wenn dessen Eingangsstufe entsprechend ausgebildet ist. Ein Beispiel hierfür ist schematisch in Fig. 9 dargestellt. Die Eingangsstufe enthält zwei Operationsverstärker 15, 16, die jeweils mit einem Eingang an eine der beiden Meßelektroden A bzw. Ref geschaltet und an ihren anderen Eingängen zusammengeschaltet sind. Die Ausgänge der beiden Operationsverstärker 15, 16 sind über je einen Widerstand R mit einem gemeinsamen Ausgang OUT3 für das Impedanzmeßsignal verbunden. Die Spannung über Z5 entspricht einer Gleichtaktspannung an den Eingängen bzw. Elektroden A und Ref der Verstärkerschaltung. Diese Gleichtaktspannung wird über die Widerstände R auf den Ausgang OUT3 geführt. Auch eine Abnahme der Meßspannung direkt am Ausgang direkt eines der beiden Operationsverstärker 15 oder 16 ist möglich, wenn das Differenzsignal zwischen A und Ref wesentlich kleiner ist als das durch den Spannungsabfall über Z5 hervorgerufene Gleichtaktsignal.

Zur Ableitung der Meßsignale werden die Schalter S11, S21, S31 und S41 geschlossen und die Schalter S12, S22, S32 und S42 (Fig. 8) geöffnet.

Die beschriebene Meßanordnung kann auf beliebig viele weitere Kanäle erweitert oder auch auf einen einzigen Kanal reduziert werden. Bei Anordnungen für zwei oder mehr Kanäle kann es von Vorteil sein, mehrere Ref-Eingänge der Verstärker an eine gemeinsame Elektrode anzuschließen, wodurch der Aufwand für die Elektrodenimpedanzmessung herabgesetzt wird. Für die zusammengeschalteten Ref-Eingänge kann eine Puffervorrichtung vorgesehen sein.

Die beschriebene Anordnung hat den Vorteil, daß die Eingangsimpedanz der als Verstärkerschaltung für die evozierten Potentiale dienenden Instrumentenverstärker im Meßbetrieb genau definiert ist und keine wesentlichen Störungen über den Generator und Eingangswiderstände in den Eingangskreis eingekoppelt werden können. Insgesamt wird durch die beschriebenen Maßnahmen ein miniaturisierbarer Vorverstärker geschaffen, der ausreichend unempfindlich gegen Störungen vom Meß-Umfeld ist.

**Patentansprüche**

1.   Verfahren zum Messen und Darstellen von reizevozierten Potentialen des Gehirns,
wobei unter Verwendung von Meßelektroden (A, Ref.), einer Verstärkerschaltung (1-6) und einer Auswerteeinrichtung (7) der zeitliche Verlauf von Informationspotentialen (MLAEP), die einer zu messenden neurophysiologischen Funktion entsprechen, dargestellt wird und zugleich von der zu messenden Funktion im wesentlichen unabhängige frühe evozierte Potentiale (BAEP) aus dem Hirnstamm gemessen werden,
dadurch gekennzeichnet, daß während der Messung und Darstellung der der Funktion entsprechenden Informationspotentiale (MLAEP) der zeitliche Verlauf der frühen evozierten Potentiale (BAEP) aus dem Hirnstamm mit einer gegenüber den Informationssignalen vergrößerten Zeitauflösung dargestellt wird.

2.   Verfahren nach Anspruch 1,
dadurch gekennzeichnet, daß die evozierenden Reize periodisch oder stochastisch verteilt wiederholt werden und jeweils nach einer vorbestimmten Zeit oder Anzahl von Meßperioden die Kurve der aktuellen Informationspotentiale dargestellt wird, welche über einen wesentlich größeren Zeitraum oder eine wesentlich größere Anzahl von vorangegangenen Meßperioden gemittelt wurde.

3.   Verfahren nach Anspruch 2,
dadurch gekennzeichnet, daß bei der Mittelung über die vorbestimmte Anzahl von Meßperioden jeweils die Mittelwerte der während einer wesentlich geringeren Zahl von Meßperioden gemessenen Signale gebildet und selbsttätig miteinander verglichen werden, und daß diese Mittelwerte ihrerseits gemittelt werden, wobei von der Mehrzahl der übrigen Mittelwerte wesentlich abweichende Mittelwerte vernachlässigt werden.

4. Verfahren nach Anspruch 2 oder 3, **dadurch gekennzeichnet,** daß bei der Überwachung der Narkosetiefe zunächst die sich beim wachen Patienten ergebenden Kurven dargestellt werden,

daß dann die sich in regelmäßigen Zeitabständen nacheinander jeweils ergebenden Kurven dargestellt werden, so daß ein Entwicklungstrend erkennbar ist,
und daß diese Kurven gleichzeitig mit den jeweils aktuellen Kurven dargestellt werden.

5. Verfahren nach dem Oberbegriff des Anspruchs 1, bei dem akustisch evozierte Potentiale mittlerer Latenz (MLAEP) als Informationspotentiale dargestellt werden, **dadurch gekennzeichnet,** daß während der Messung der reizevozierten Potentiale der zeitliche Verlauf von Änderungen eines charakteristischen Peaks ($N_b$) der MLAEP-Kurve und/oder dessen Latenz und/oder des von der Auswerteeinrichtung (7) errechneten Quotienten aus diesen beiden Faktoren dargestellt wird.

6. Meßanordnung zum Messen und Darstellen von reizevozierten Potentialen des Gehirns,
mit Meßelektroden (A, Ref), zur Ableitung der reizevozierten Potentiale, einer Verstärkerschaltung (1-6), und einer einen Rechner enthaltenden Auswerteeinrichtung (7), zur Darstellung des zeitlichen Verlaufs von Informationspotentialen (MLAEP), die einer zu messenden neurophysiologischen Funktion entsprechen, mit einer bestimmten Zeitauflösung, und zur Darstellung von frühen evozierten Potentialen (BAEP), aus dem Hirnstamm, die von der zu messenden Funktion im wesentlichen unabhängig sind,
**dadurch gekennzeichnet,** daß die Auswerteeinrichtung (7), so ausgebildet ist, daß während der Messung und Darstellung der der Funktion entsprechenden Informationspotentiale (MLAEP) der zeitliche Verlauf der frühen evozierten Potentiale (BAEP) aus dem Hirnstamm mit einer gegenüber der bestimmten Zeitauflösung vergrößerten Zeitauflösung dargestellt wird.

7. Meßanordnung nach Anspruch 6, **dadurch gekennzeichnet,** daß die Potentiale (BAEP) aus dem Hirnstamm mit einer gegenüber den Informationspotentialen (MLAEP) vergrößerten Amplitudenauflösung dargestellt werden.

8. Meßanordnung nach Anspruch 6 oder 7, **dadurch gekennzeichnet,** daß der Verlauf der frühen akustisch evozierten Potentiale aus dem Hirnstamm (BAEP) neben der Informationskurve dargestellt wird.

9. Meßanordnung nach einem der Ansprüche 6 - 8, **dadurch gekennzeichnet,** daß gemeinsam mit dem zeitlichen Verlauf der Meßwerte der zeitliche Verlauf eines von der Auswerteeinrichtung (7) errechneten veränderlichen Meßgütefaktors dargestellt wird.

10. Meßanordnung nach einem der Ansprüche 6 - 9, **dadurch gekennzeichnet,** daß in Verbindung mit den dargestellten Kurven selbsttätig errechnete Zeit- und/oder Amplitudenmarkierungen und/oder Zahlenwerte angezeigt und/oder dargestellt werden.

11. Meßanordnung nach Anspruch 6 zum Messen von reizevozierten Potentialen des Gehirns,

mit einer Elektrodenanordnung (A, Ref) zur Ableitung der reizevozierten Potentiale,
mit einer Auswerteeinheit (7) für die Auswertung und/oder Darstellung der Meßsignale,
und mit einer zwischen die Elektrodenanordnung (A,Ref) und die Auswerteeinheit (7) geschalteten Verstärkerschaltung, die die reizevozierten Potentiale des Gehirns auf die für die Auswertung erforderlichen Spannungen verstärkt und eine Eingangsstufe (1), eine Anzahl von weiteren Verstärkerstufen (3,5) und eine Trennstufe (8) enthält, die die von einer der Verstärkerstufen (3) abgegebenen reizevorzierten Potentiale überträgt und die Elektrodenanordnung (A,Ref) galvanisch von der Auswerteeinheit (7) entkoppelt,

**dadurch gekennzeichnet,** daß die Gesamtverstärkung des zwischen die Elektrodenanordnung (A,Ref) und die Trennstufe (8) geschalteten Verstärkerteils (1,3) wesentlich größer als 100, jedoch geringer als $10^4$ ist und für die restliche Verstärkung der reizevorzierten Potentiale ein zwischen die Trennstufe (8) und die Auswerteeinheit (7) geschalteter Nachverstärker (5) vorgesehen ist.

12. Meßanordnung nach Anspruch 11, **dadurch gekennzeichnet,** daß die Gesamtverstärkung des zwischen die Elektrodenanordnung (A,Ref) und die Trennstufe (8) geschalteten Verstärkerteils (1,3) zwischen ungefähr 1000 und 4000 beträgt.

13. Meßanordnung nach Anspruch 11 oder 12, **dadurch gekennzeichnet,** daß eine Instrumentenverstärkerstufe als Eingangsstufe (1), ein Hochpaß (2) und eine Mittenverstärkerstufe (3) der Reihe nach zwischen die Elektroden (A,Ref) und die Trennstufe (8) und ein Tiefpaß (4), ein Nachverstärker (5) und ein A/D-Wandler (6) der Reihe nach zwischen die Trennstufe (8) und die Auswerteeinheit (7) geschaltet sind.

14. Meßanordnung nach einem der Ansprüche 11 - 13, **dadurch gekennzeichnet,** daß die mit je einer Meßelektrode (A,Ref) verbundenen und über je einen Eingangsabschlußwiderstand (R1...R4) an Bezugspotential (Masse) geschalteten Eingänge der Verstärkerschaltung an eine einen Meßstromgenerator (12) enthaltende Impedanzmeßschaltung zum Messen der Elektrodenimpedanzen angeschlossen sind.

15. Meßanordnung nach Anspruch 14, **dadurch gekennzeichnet,** daß die Impedanzmeßschaltung je einen zwischen den Eingangsabschlußwiderstand (R1...R4) an jeder Meßelektrode (A1...Ref2) und das Bezugspotential geschalteten ersten Schalter (11...S41)
und je einen zwischen die Verbindung des Elektrodenabschlußwiderstands (R1...R4) mit dem ersten Schalter (S11...S41) und dem Meßstromgenerator (12) geschalteten zweiten Schalter (S12...S42) enthält.

16. Meßanordnung nach Anspruch 15, **dadurch gekennzeichnet,** daß die Verstärkerschaltung den Spannungsabfall an der Elektrodenimpedanz mißt, der von dem bei geöffnetem ersten Schalter (S11...S41) und geschlossenem zweiten Schalter (S12...S42) durch die Elektrodenimpedanz fließenden Strom des Meßstromgenerators (12) erzeugt wird.

17. Meßanordnung nach Anspruch 15 oder 16, **dadurch gekennzeichnet,** daß der Meßstromgenerator (12) über je einen der zweiten Schalter (S12...S42) selektiv mit jeder der Elektrodenimpedanzen am Eingang von zwei oder mehr Verstärkerschaltungen (10,11) koppelbar ist, die jeweils an eine aktive Meßelektrode (A1,A2) und eine Referenzelektrode (Ref1, Ref2) angeschlossen sind.

18. Meßanordnung nach einem der Ansprüche 15 - 17, **dadurch gekennzeichnet,** daß zum Messen der Impedanz einer an das Bezugspotential (Masse) angeschlossenen Elektrode (Gnd) ein weiterer Schalter (S6) vorgesehen ist, der zwischen einen Verstärkereingang und das Bezugspotential geschaltet ist, und/oder ein parallel zu der Verstärkerschaltung (11) geschalteter gesonderter Impedanzmeßverstärker (13) vorgesehen ist,
und/oder die Eingangsstufe der Verstärkerschaltung zwei Operationsverstärker (15,16) enthält, die jeweils mit einem Eingang an eine der beiden Meßelektroden (A,Ref) geschaltet und an ihren anderen Eingängen zusammengeschaltet sind und einen Ausgang (OUT3) für das Impedanzmeßsignal haben.

**Claims**

1. Method of measuring and displaying evoked cerebral response potentials wherein the pattern over time of information potentials (MLAEP), which correspond to a neurophysiological function to be measured, is displayed using measuring electrodes (A,Ref.), an amplifier circuit (1-6) and an evaluation circuit (7), and at the same time early evoked potentials (BAEP) from the brain stem, substantially independent of the function to be measured, are measured, characterised in that during the measuring and display of the information potentials (MLAEP) corresponding to the function the pattern over time of the early evoked potentials (BAEP) from the brain stem are displayed with an increased time resolution with respect to the information potentials.

2. Method as claimed in claim 1, characterised in that the evoking stimuli are repeated periodically or stochastically distributed and after a predetermined time or number of measurement periods the curve of the current information potentials is displayed which was averaged over a substantially greater period of time or a substantially greater number of preceding measurement periods.

3. Method as claimed in claim 2, characterised in that when averaging over the predetermined number of measuring periods, the average values of the signals measured during a substantially smaller number of measuring periods are formed and automatically compared with one another and that these average values are in turn averaged, whereby average values deviating substantially from the other average values are ignored.

4. Method as claimed in claim 2 or 3, characterised in that when monitoring the depth of narcosis, the curves produced with a wakeful patient are firstly displayed, that the curves produced at regular time intervals are then successively displayed so that a development trend may be detected and that these curves are displayed at the same time as the current curve.

5. Method as claimed in the preamble of claim 1 in which acoustically evoked potentials of mid-latency (MLAEP) are displayed as information potentials, characterised in that during the measurement of the evoked response potentials the pattern of changes over time of a characteristic peak ($N_b$) of the MLAEP curve and/or its latency and/or the quotient of these two factors calculated by the evaluation device (7) is displayed.

6. Measuring system for measuring and displaying evoked cerebral response potentials including measuring electrodes (A, Ref.) for picking up the evoked response potentials, an amplifier circuit (1-6) and an evaluation device (7) including a computer for displaying the pattern over time of information potentials (MLAEP), which correspond to a neurophysiological function to be measured, with a predetermined time resolution, and for displaying early evoked potentials (BAEP) from the brain stem, which are substantially independent of the function to be measured, characterised in that the evaluation device (7) is so constructed that during the measurement and display of the information potentials (MLAEP) corresponding to the function the pattern over time of the early evoked potentials (BAEP) from the brain stem is displayed with a time resolution which is increased with respect to the predetermined time resolution.

7. Measuring system as claimed in claim 6, characterised in that the potentials (BAEP) from the brain stem are displayed with an amplitude resolution which is increased with respect to the information potentials (MLAEP).

8. Measuring system as claimed in claim 6 or 7, characterised in that the pattern of the early acoustically evoked potentials from the brain stem (BAEP) is displayed in addition to the information curve.

9. Measuring system as claimed in one of claims 6-8, characterised in that, together with the pattern over time of the measured values, the pattern over time of a variable measuring quality factor calculated by the evaluation device (7) is displayed.

10. Measuring system as claimed in one claims 6-9, characterised in that in conjunction with the displayed curves automatically calculated time and/or amplitude markings and/or numerical values are indicated and/or displayed.

11. Measuring system as claimed in claim 6 for measuring evoked cerebral response potentials including an electrode arrangement (A, Ref.) for tapping off the evoked response potentials, an evaluation unit (7) for evaluating and/or displaying the measured signals and an amplifier circuit, which is connected between the electrode arrangement (A, Ref.) and the evaluation unit (7) and amplifies the evoked cerebral response potentials to the voltages necessary for evaluation and includes an input stage (1), a number of further amplifier stages (3,5) and an isolation stage (8), which transmits the evoked response potentials delivered by one of the amplifier stages (3) and galvanically decouples the electrode arrangement (A, Ref.) from the evaluation unit (7), characterised in that the total amplification of the amplifier portion (1,3) connected between the electrode arrangement (A, Ref.) and the isolating stage (8) is substantially larger than 100 but less than $10^4$ and a post amplifier (5), connected between the isolation stage (8) and the evaluation unit (7), is provided for the remaining amplification.

12. Measuring system as claimed in claim 11, characterised in that the total amplification of the amplifier portion (1,3) connected between the electrode arrangement (A, Ref.) and the isolation stage (8) is between about 1000 and 4000.

13. Measuring system as claimed in claim 11 or 12, characterised in that an instrument amplifier stage constituting an input stage (1), a high-pass filter (2) and a centre amplifier stage (3) are connected serially between the electrodes (A, Ref) and the isolation stage (8) and a high-pass filter (4), a post amplifier (5) and a A/D converter (6) are connected serially between the isolation stage (8) and the evaluation unit (7).

14. Measuring system as claimed in one of claims 11-13, characterised in that the inputs of the amplifier circuit, which are connected to a respective measuring electrode (A,Ref) and are connected via a respective input terminal resistance (R1...R4) to a reference potential (earth), are connected to an impedance measurement circuit, which includes a measuring current generator (12), for measuring the electrode impedances.

15. Measuring system as claimed in claim 14, characterised in that the impedance measuring circuit includes a respective first switch (11...S41) connected between the input terminal resistance (R1...R4) on each measuring electrode (A1....Ref2) and the reference potential and a respective second switch (S12...S42) connected between the connection of the electrode terminal resistance (R1...R4) with the first switch (S11...S41) and the measuring current generator (12).

16. Measuring system as claimed in claim 15, characterised in that the amplifier circuit measures the voltage drop at the electrode impedance which is produced by the current from the measuring current generator (12) flowing through the electrode impedance when the first switch (S11...S41) is open and the second switch (S12...S42) is closed.

**17.** Measuring system as claimed in claim 15 or 16, characterised in that the measuring current generator (12) may be coupled via a respective one of the second switches (S12...S42) selectively to each of the electrode impedances at the input of two or more amplifier circuits (10,11) which are each connected to an active measuring electrode (A1,A2) and a reference electrode (Ref1,Ref2).

**18.** Measuring system as claimed in one of claims 15-17, characterised in that for the purpose of measuring the impedance of an electrode (Gnd) connected to the reference potential (earth), a further switch (S6) is provided, which is connected between an amplifier input and the reference potential, and/or a separate impedance measurement amplifier (13) is provided connected in parallel to the amplifier circuit (11), and/or the input stage of the amplifier circuit includes two operational amplifier (15,16) which are each connected with one input to one of the two measuring electrodes (A Ref) and are connected together at their other inputs and have an output (OUT 3) for the impedance measurement signal.

**Revendications**

**1.** Procédé de mesure et de représentation de potentiels cérébraux évoqués par stimulation dans lequel, par utilisation d'électrodes de mesure (A, Ref.), d'un circuit d'amplification (1 à 6) et d'un dispositif d'exploitation (7), la variation dans le temps de potentiels d'information (MLAEP) qui correspondent à une fonction neurophysiologique à mesurer est représentée et en même temps des potentiels précoces évoqués (BAEP) provenant du cerveau et essentiellement indépendants de la fonction à mesurer, sont mesurés caractérisé en ce que pendant la mesure et la représentation des potentiels d'information (MLAEP), correspondant à la fonction, la variation dans le temps des potentiels précoces évoqués (BAEP) provenant du cerveau est représentée avec une résolution de temps augmentée par rapport aux potentiels d'information.

**2.** Procédé selon la revendication 1, caractérisé en ce que les stimulations évocatrices sont renouvelées, distribuées périodiquement ou de manière aléatoire et dans chaque cas, après un temps prédéterminé ou un nombre prédéterminé de périodes de mesure, il est représenté la courbe des potentiels d'information actuels qui a été déterminée sur un laps de temps sensiblement plus grand ou un nombre sensiblement plus grand de périodes de mesure précédentes.

**3.** Procédé selon la revendication 2, caractérisé en ce que pour former la moyenne sur le nombre prédéterminé de périodes de mesure, on forme les valeurs moyennes des signaux mesurés pendant un nombre sensiblement inférieur de périodes de mesure et on les compare automatiquement entre elles et en ce qu'on fait la moyenne de ces valeurs moyennes, les valeurs moyennes qui s'écartent sensiblement de la majorité des autres valeurs moyennes étant négligées.

**4.** Procédé selon la revendication 2 ou 3, caractérisé en ce que lors du contrôle de la profondeur de l'anesthésie, sont représentées d'abord les courbes obtenues lorsque le patient est éveillé,

en ce qu'ensuite sont représentées les courbes que l'on obtient l'une après l'autre à intervalles de temps réguliers, de sorte que l'on peut déceler une tendance évolutive,

et en ce que ces courbes sont représentées en même temps que les courbes actuelles.

**5.** Procédé selon la revendication 1, dans lequel des potentiels évoqués acoustiquement de latence moyenne (MLAEP) sont représentés en tant que potentiels d'information, caractérisé en ce que pendant la mesure des potentiels évoqués par stimulation est représentée la courbe de temps des variations d'un pic caractéristique ($N_b$) de la courbe (MLAEP) et/ou de sa latence et/ou du quotient calculé par le dispositif d'exploitation (7) à partir de ces deux facteurs.

**6.** Dispositif de mesure de potentiels cérébraux évoqués par stimulation, comportant des électrodes de mesure (A, Ref) pour dériver des potentiels évoqués par stimulation, un circuit d'amplification (1 à 6) ainsi qu'un dispositif d'exploitation (7) contenant un ordinateur pour la représentation de la variation dans le temps de potentiels d'information (MLAEP), qui correspondent à une fonction neurophysiologique à mesurer, avec une résolution de temps déterminée et pour la représentation de potentiels précoces évoqués (BAEP) provenant du cerveau, qui sont sensiblement indépendants de la fonction à mesurer, caractérisé en ce que le dispositif d'exploitation (7) est réalisé de manière que pendant la mesure et la représentation des potentiels d'information (MLAEP) correspondant à la fonction, est représentée la variation dans le temps des potentiels précoces évoqués (BAEP) provenant du cerveau, avec une résolution de temps augmentée par rapport à la résolution de temps déterminée.

**7.** Dispositif de mesure selon la revendication 6, caractérisé en ce que les potentiels (BAEP) provenant du cerveau sont représentés avec une résolution d'amplitude augmentée par rapport aux potentiels d'information (MLAEP).

8. Dispositif de mesure selon la revendication 6 ou 7, caractérisé en ce que la variation des potentiels précoces évoqués acoustiquement provenant du cerveau (BAEP) est représentée à côté de la courbe d'information.

9. Dispositif de mesure selon la revendication 6 à 8, caractérisé en ce qu'avec la variation dans le temps des valeurs de mesure est représentée la variation dans le temps d'un facteur de qualité de mesure variable, calculé par le dispositif d'exploitation (7).

10. Dispositif de mesure selon l'une des revendications 6 à 9, caractérisé en ce que sont affichés et/ou représentés des marquages de temps et/ou d'amplitude et/ou des valeurs chiffrées, calculées automatiquement en combinaison avec les courbes représentées.

11. Dispositif de mesure selon la revendication 6 pour la mesure de potentiels cérébraux évoqués par stimulation,

comportant un dispositif à électrode (A, Rets, pour la dérivation des potentiels évoqués par stimulation,

comportant une unité d'exploitation (7) pour l'exploitation et/ou la représentation des signaux de mesure,

et comportant un circuit d'amplification monté entre le dispositif à électrode (A, Ref) et l'unité d'exploitation (7), qui amplifie les potentiels cérébraux évoqués par stimulation aux tensions nécessaires à l'exploitation et qui contient un étage d'entrée (1), un nombre d'autres étages d'amplification (3, 5) et un étage de séparation (8) qui transmet les potentiels évoqués par stimulation, fournis par l'un des étages d'amplification (3), et qui désaccouple galvaniquement le dispositif à électrode (A, Ref) de l'unité d'exploitation (7),

caractérisé en ce que l'amplification totale de la partie d'amplification (1, 3) montée entre le dispositif (A, Ref) et l'étage de séparation (8) est sensiblement supérieure à 100 mais inférieure à $10^4$ et pour l'amplification restante des potentiels évoqués par stimulation, un amplificateur complémentaire (5) est prévu entre l'étage de séparation (8) et l'unité d'exploitation (7).

12. Dispositif de mesure selon la revendication 11, caractérisé en ce que l'amplification totale de la partie d'amplification (1, 3) montée entre le dispositif à électrode (A, Ref) et l'étage de séparation (8) est comprise entre 1 000 et 4 000 environ.

13. Dispositif de mesure selon la revendication 11 ou 12, caractérisé en ce que sont montés dans l'ordre un étage d'amplification d'instrument servant d'étage d'entrée (1), un passe-haut (2) et un étage d'amplification central (3), entre les électrodes (A, Ref) et l'étage de séparation (8), et sont montés dans l'ordre un passe-bas (4), un amplificateur complémentaire (5) et un convertisseur analogique/numérique (6) entre l'étage de séparation (8) et l'unité d'exploitation (7).

14. Dispositif de mesure selon l'une des revendications 11 à 13, caractérisé en ce que les entrées du circuit d'amplification, reliées chacune à une électrode de mesure (A, Ref) et reliées au potentiel de référence (masse) chacune par une résistance de fermeture d'entrée (R1...R4), sont raccordés à un circuit de mesure d'impédance, contenant un générateur de courant de mesure (12), pour la mesure des impédances des électrodes.

15. Dispositif de mesure selon la revendication 14, caractérisé en ce que le circuit de mesure d'impédance contient

un premier interrupteur (11...S41) monté entre la résistance de fermeture d'entrée (R1...R4) à chaque électrode de mesure (A1...Ref2) et le potentiel de référence

et un deuxième interrupteur (S12..S42) monté entre la liaison de la résistance de fermeture électrode (R1...R4) avec le premier interrupteur (S11...S41) et le générateur de courant de mesure (12).

16. Dispositif de mesure selon la revendication 15, caractérisé en ce que le circuit d'amplification mesure la chute de tension sur l'impédance d'électrode, qui est produite par le courant du générateur de courant de mesure (12) qui traverse l'impédance d'électrode, lorsque le premier interrupteur (S11...S41) est ouvert et le deuxième interrupteur (S12...S42) est fermé.

17. Dispositif de mesure selon la revendication 15 ou 16, caractérisé en ce que le générateur de courant de mesure (12) peut être couplé, par l'un des deuxièmes interrupteurs (S12...S42), de manière sélective, avec chacune des impédances d'électrodes, à l'entrée de deux circuits d'amplification (10, 11) ou plus, lesquels sont raccordés chacun à une électrode de mesure active (A1, A2) et à une électrode de référence (Ref1, Ref2).

18. Dispositif de mesure selon l'une des revendications 15 à 17, caractérisé en ce que pour mesurer l'impédance d'une électrode (Gnd) raccordée au potentiel de référence (masse), il est prévu un autre interrupteur (S6) qui est monté entre une entrée d'amplificateur et le potentiel de référence et/ou il est prévu un amplificateur de mesure d'impédance (13) séparé monté parallèlement au circuit d'amplification (11),
et/ou l'étage d'entrée du circuit d'amplification contient deux amplificateurs opérationnels (15, 16), qui sont montés chacun par une entrée à l'une des deux électrodes de mesure (A, Ref) et qui sont reliés par leurs autres entrées et présentent une entrée (out3) pour le signal de mesure d'impédance.

# Fig. 1

# Fig. 9

Fig. 2

Fig. 3

Fig. 4

# Fig. 5

EP 0 813 840 B1

Fig.6

Fig. 7

A

Ref

Gnd

1  2  3  8  4  5  6  7

OUT

Fig. 8

Z1  A1

Z2  Ref1

Z3  A2

Z4  Ref2

Z5  Gnd

R1  R2  R3  R4

S11  S21  S31  S41

S12

S22

S32

S42

Rx  U₀

Ry

12  ein/aus

OUT1  10

OUT2  11

OUT3  13

S6

EP 0 813 840 B1

21